# EUROPEAN PATENT SPECIFICATION

(11) **EP 0 932 380 B1**
(45) Date of publication and mention of the grant of the patent: **12.06.2002**
(21) Application number: 97909936.3
(22) Date of filing: 03.10.1997
(51) Int. Cl.: A61F 13/15

(54) **COMBINATION ABSORBENT ARTICLE**
KOMBINIERTER ABSORBTIONSARTIKEL
COMBINAISON D'ARTICLES ABSORBANTS

(30) Priority: 21.10.1996 US 734679
(43) Date of publication of application: 04.08.1999
(73) Proprietor: THE PROCTER & GAMBLE COMPANY, Cincinnati, Ohio 45202 (US)
(72) Inventor: MAYER, Katherine, Louise, Newport, KY 41071 (US)
(74) Representative: Kohol, Sonia
(86) International application number: US9717797
(87) International publication number: WO9817220

(56) References cited:
- EP-A- 0 688 548
- EP-A- 0 788 785
- WO-A-95/29655
- WO-A-97/16147
- US-A- 2 683 457
- US-A- 2 929 379
- US-A- 5 429 631
- US-A- 5 599 339

## Description

### FIELD OF THE INVENTION

This invention is a combination absorbent article which comprises at least a first absorbent article and a second absorbent article that are releasably secured together only by mechanical securement means. No adhesive is used or required to secure the two absorbent articles together.

### BACKGROUND OF THE INVENTION

Various absorbent articles such as sanitary napkins and light to medium incontinent devices exist which absorb body exudates such as menses, urine and fecal matter. Disposable products of this type generally comprise a fluid permeable topsheet, a fluid absorbent core, and a fluid impermeable backsheet. Various shapes, sizes and thicknesses of such articles have been explored in an attempt t0 make their use more comfortable and convenient. For example. U.S. Patent No. 5.389.094, issued to Lavash et al., on February 14, 1995, U.S. Patent No. 5,383,869, issued to Osborn, III, on January 24, 1995, U. S. Patent No. 5,382,245, issued to Thompson et al.. on January 17, 1995 and U.S. Patent No. 5,346,486, issued to Osborn III. et al. on September 13, 1994 show numerous shapes, sizes, thicknesses and other alternative variations.

Additionally, combination or multiple absorbent articles have been explored in an attempt to provide one or more readily available clean, fresh absorbent articles for use once a previous article has been fully used and requires disposal. In some embodiments, a plurality of absorbent articles have been stacked on top of each other and placed in a user's undergarment in an attempt to provide readily available, additional absorbent articles. Also, these articles have generally been sealed to one-another in a number of ways. One method includes stacking the absorbent articles and then sealing them to one-another about their peripheries with an adhesive. For example, U.S. Patent No. 5,429,631 issued to Grenier on July 4, 1995, discloses a combination absorbent article which uses an adhesive to secure one absorbent article to another absorbent article. U.S. Patent No. 4,505,707 issued to Feeney on March 19, 1985 discloses a multiple absorbent article that has each pad being bonded to an underlying pad by spot adhesives or heat compression.

WO 95/29655 describes multilayered absorbent feminine hygiene products which are attached through adhesive means, thermal and/or compressive bonding means or mechanical means.

There are certain drawbacks associated with using adhesives to secure multiple absorbent articles together. Adhesives, because of their adherent qualities, can be contaminated by foreign materials from numerous sources including the air or a user's garment or can themselves be a source of irritation if they come in contact with a user's skin, especially near the user's pudendal region. By not using adhesives, one can avoid the possibility of having a contaminated and unsanitary absorbent article due to adhesive present on the body-contacting surface of an absorbent article. Therefore, it would be desirable to construct a combination absorbent article which does not use any adhesive to secure the various articles together within the combination article.

To these ends, applicants have provided a combination absorbent article that joins a plurality of absorbent articles by mechanical securement means located along the peripheral edges of the articles. Thus, a user can have the convenience of a combination absorbent article that is free from the problems associated with adhesives.

### SUMMARY OF THE INVENTION

Accordingly, the present invention provides a combination absorbent article, comprising a first absorbent article having a topsheet, a backsheet joined to the topsheet, an absorbent core positioned between the topsheet and the backsheet, a body surface and a garment surface and a periphery comprising a pair of first longitudinal edges and a pair of first end edges. Also, the combination absorbent article comprises a second absorbent article having a topsheet, a backsheet joined to the topsheet, an absorbent core positioned between the topsheet and the backsheet, a body or article contacting surface and a garment surface, and a periphery comprising a pair of second longitudinal edges and a pair of second end edges. The first longitudinal edges of the first absorbent article and the second longitudinal edges of the second absorbent article are releasably secured together only by mechanical securement means, preferably comprising crimps/embossments along the first and second longitudinal edges; thus, a combination absorbent article is formed from the securement of the first and second absorbent articles along their first and second longitudinal edges.

The combination absorbent article also comprises a pair of flaps joined to the first absorbent article and a pair of flaps joined to the second absorbent article. The flaps are disposed adjacent to and extend laterally outwardly from each of the first and second longitudinal edges; i.e., there is at least one flap adjacent to and joined to each longitudinal edge.

Each flap comprises mechanical securement means whereby the flaps on the first and second absorbent articles are releasably secured together.

To facilitate enhanced cohesion between the mechanically secured or crimped portions of the first and second absorbent articles, at least the periphery of the second absorbent article comprises a nonwoven layer joined to the article contacting surface thereof. In this state, when the peripheries and the flaps of the first and second absorbent articles are crimped together, i.e., releasably mechanically secured together, their cohesion strength can be greater than if they were crimped together without a nonwoven layer. This nonwoven layer will preferably also be joined to the article contacting surface of the flaps.

### BRIEF DESCRIPTION OF THE DRAWINGS

While the specification concludes with claims particularly pointing out and distinctly claiming the subject matter which is regarded as forming the present invention, it is believed that the invention will be better understood from the following descriptions which are taken in conjunction with the accompanying drawings in which like designations are used to designate substantially identical elements, and in which:
Figure 1 is an exploded view of an absorbent article not forming part of the present invention having portions cut away to reveal underlying structure;
Figure 2 is a plan view of an absorbent article of the present invention having flaps and mechanical securement means comprised on the flaps;
Figure 3 is a side cut view of crimps being spaced apart;
Figure 3A is a side cut view of the crimps of Fig. 3 wherein the crimps are in operable relationship to one another; and
Figure 4 is a plan view of a preferred embodiment of a SELF web having a strainable network of the present invention with the rib-like elements facing toward the viewer.

### DETAILED DESCRIPTION OF THE INVENTION

As used herein, the term "absorbent article" refers to devices which absorb and contain body exudates, and, more specifically, refers to devices which are placed against or in proximity to the body of the wearer to absorb and contain the various exudates discharged from the body. The terms "mechanical securement means" or "releasably mechanically securement means" are used herein to describe a means by which two or more items are releasably secured together by mechanical engagement of the items to one another. The terms "mechanical securement", "mechanical engagement", "mechanically secured" or "mechanically engaged" are used herein to refer to two or more absorbent articles which are joined (i.e., releasably mechanically secured) to one-another without the use of adhesive. By the terms "crimp", or "embossment" it is meant herein to refer to folds or pleats along the longitudinal edges and/or end edges of the first and second absorbent articles herein. The term "disposable" is used herein to describe absorbent articles which are not intended to be laundered or otherwise restored or reused as an absorbent article (i.e., they are intended to be discarded after a single use, and, preferably, to be recycled, composted or otherwise disposed of in an environmentally compatible manner). As used herein, the term "sanitary napkin" or "napkin" refers to devices which absorb and contain body exudates, and more specifically, refers to an absorbent article which is worn by females adjacent to the pudendal region, generally external to the urogenital region, and which is intended to absorb and contain menstrual fluids and other vaginal discharges from the wearer's body (e.g., blood, menses, and urine). As used herein, the term "pudendal" refers to the externally visible female genitalia. It should be understood, however, that the present invention is also applicable to other feminine hygiene garments or catamenial pads such as pantiliners or other absorbent articles such as diapers, incontinence pads, and the like.

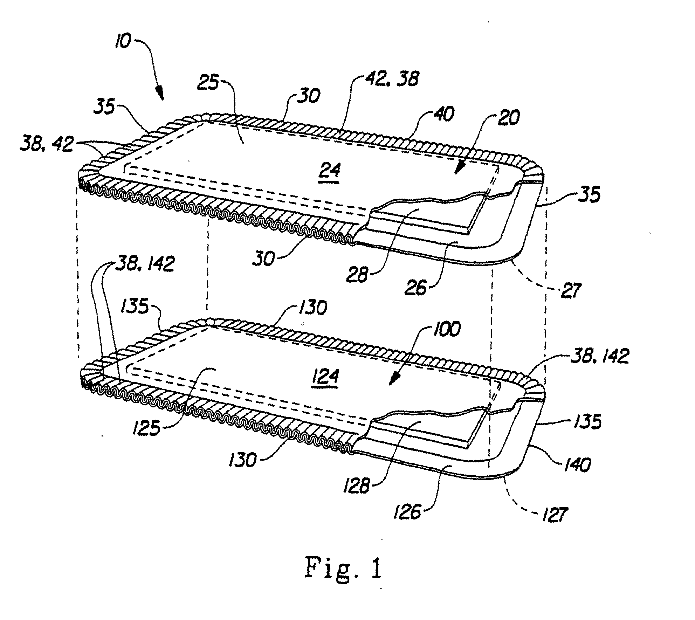

Figure 1 is an exploded perspective view of a combination absorbent article 10 not forming part of the present invention. The combination absorbent article 10 comprises a first absorbent article 20 and a second absorbent article 100. In the embodiment shown in Figure 1, the first absorbent article 20 and the second absorbent article 100 have the same width and length dimensions.

A first absorbent article 20 is shown in Figure 1 with portions of the structure being cut-away to more clearly show the construction of the first absorbent article 20. The first absorbent article 20 comprises a liquid pervious topsheet 24, a liquid impervious backsheet 26 joined with the topsheet 24, and an absorbent core 28 positioned between the topsheet 24 and the backsheet 26.

The first absorbent article 20 has two surfaces, a body-contacting surface or "body surface" 25 and a garment surface 27 (not shown). The body surface 25 is intended to be worn adjacent to the body of the wearer while the garment surface 27 is on the opposite side and is intended to be placed adjacent to the second absorbent article 100. Figure 1 also shows that the first absorbent article 20 has a periphery 40 which comprises longitudinal edges 30 and end edges 35.

While the topsheet, the backsheet, and the absorbent core may be assembled in a variety of well known absorbent article configurations (including so called "tube" products or side flap products), preferred absorbent article configurations are described generally in U.S. Patent 4,950,264, "Thin, Flexible Sanitary Napkin" issued to Osborn on August 21, 1990; U.S. Patent 4,321,924, "Bordered Disposable Absorbent Article" issued to Ahr on March 30, 1982; U.S. Patent 4,589,876, "Shaped Sanitary Napkin With Flaps" issued to Van Tilburg on May 20, 1986. Figure 1 shows an embodiment of the first absorbent article 20 in which the topsheet 24 and the backsheet 26 have length and width dimensions generally larger than those of the absorbent core 28. The topsheet 24 and the backsheet 26 extend beyond the edges of the absorbent core 28 to form portions of the periphery 40.

The absorbent core 28 may be any absorbent means which is capable of absorbing or retaining liquids (e.g., menses and/or urine). As shown in Figure 1 the absorbent core 28 has a body surface, a garment surface, side edges, and end edges. The absorbent core 28 may be manufactured in a wide variety of sizes and shapes (e.g., rectangular, oval, hourglass, dog bone, asymmetric, etc.) and from a wide variety of liquid-absorbent materials commonly used in sanitary napkins and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these. The configuration and construction of the absorbent core 28 may also be varied (e.g., the absorbent core 28 may have varying caliper zones (e.g., profiled so as to be thicker in the center), hydrophilic gradients, superabsorbent gradients, or lower density and lower average basis weight acquisition zones; or may comprise one or more layers or structures). The total absorbent capacity of the absorbent core 28 should, however, be compatible with the design loading and the intended use of the first absorbent article 20. Further, the size and absorbent capacity of the absorbent core 28 may be varied to accommodate different uses such as incontinence pads, pantiliners, regular sanitary napkins, or overnight sanitary napkins.

Exemplary absorbent structures for use as the absorbent core 28 of the present invention are described in U.S. Patent 4,950,264 entitled "Thin, Flexible Sanitary Napkin" issued to Osborn on August 21, 1990; U.S. Patent 4,610,678 entitled "High-Density Absorbent Structures" issued to Weisman et al. on September 9, 1986; U.S. Patent 4,834,735 entitled "High Density Absorbent Members Having Lower Density and Lower Basis Weight Acquisition Zones", issued to Alemany et al. on May 30, 1989; and European Patent Application No. 0 198 683, The Procter & Gamble Company, published October 22, 1986 in the name of Duenk, et al.

The backsheet 26 and the topsheet 24 are positioned adjacent the garment surface and the body surface, respectively, of the absorbent core 28 and are preferably joined thereto and to each other by attachment means (not shown) such as those well known in the art. For example, the backsheet 26 and/or the topsheet 24 may be secured to the absorbent core 28 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul. Minnesota under the designation HL-1258 or H-2031. The attachment means will preferably comprise an open pattern network of filaments of adhesive as is disclosed in U.S. Patent 4,573,986 entitled "Disposable Waste-Containment Garment", which issued to Minetola, et al. on March 4, 1986.

An exemplary attachment means of an open pattern network of filaments comprises several lines of adhesive filaments swirled into a spiral pattern such as illustrated by the apparatus and method shown in U.S. Patent 3,911,173 issued to Sprague, on October 7, 1975; U.S. Patent 4,785,996 issued to Ziecker, et al. on November 22, 1978; and U.S. Patent 4.842,666 issued to Werenicz on June 27, 1989.

Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the absorbent articles described herein such as pants, pajamas and undergarments. The backsheet 26 may thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils). Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet 26 may permit vapors to escape from the absorbent core 28 (i.e., breathable) while still preventing exudates from passing through the backsheet 26.

The topsheet 24 is compliant, soft feeling, and non-irritating to the wearer's skin. Further, the topsheet 24 is liquid pervious permitting liquids (e.g., menses and/or urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. A preferred topsheet comprises an apertured formed film. Apertured formed films are preferred for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent 3,929,135, entitled "Absorptive Structures Having Tapered Capillaries", which issued to Thompson on December 30, 1975; U.S. Patent 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel. et al. on August 3, 1982; U.S. Patent 4,463,045 entitled "Macroscopically Expanded Three-Dimensional Plastic Web Exhibiting Non-Glossy Visible Surface and Cloth-Like Tactile Impression", which issued to Ahr et al. on July 31, 1984; and U.S. 5,006,394 "Multilayer Polymeric Film" issued to Baird on April 9, 1991. The preferred topsheet for the present invention is the formed film described in one or more of the above patents and marketed on sanitary napkins by The Procter & Gamble Company of Cincinnati, Ohio as "DRI-WEAVE".

In a preferred embodiment of the present invention, the body surface of the formed film topsheet is hydrophilic so as to help liquid to transfer through the topsheet faster than if the body surface was not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in PCT. Patent Application WO 93/09741, "Absorbent Article Having A Nonwoven and Apertured Film Coversheet". Alternatively, the body surface of the topsheet can be made hydrophilic by treating it with a surfactant such as is described in the above referenced U.S. 4,950,264 issued to Osborn.

The second absorbent article 100 comprises a liquid pervious topsheet 124, a liquid impervious backsheet 126 secured to the topsheet 124, and an absorbent core 128 positioned between the topsheet 124 and the backsheet 126.

The second absorbent article 100 has two surfaces, an absorbent article-contacting surface 125 which becomes a "body surface" at the removal of the first absorbent article 20 and a garment surface 127 (not shown). The second absorbent article 100 is shown in Figure 1 with portions of the structure being cut-away to more clearly show the construction of the second absorbent article 100. The absorbent article-contacting surface 125 is intended to be placed adjacent to the garment surface 27, or backsheet 26 of the first absorbent article 20. Upon removal of the first absorbent article 20 from a wearer's undergarment, the absorbent article-contacting surface 125 of the second absorbent article 100 will become the body-contacting surface 125 and will thus be worn adjacent to the wearer's body. Figure 1 also shows that the second absorbent article 100 has a periphery 140 which comprises longitudinal edges 130 and end edges 135.

While the topsheet, the backsheet, and the absorbent core may be assembled in a variety of well known absorbent article configurations (including so called "tube" products or side flap products), preferred absorbent article configurations are described generally in U.S. Patent 4,950,264, "Thin, Flexible Sanitary Napkin" issued to Osborn on August 21, 1990; U.S. Patent 4,321,924, "Bordered Disposable Absorbent Article" issued to Ahr on March 30, 1982; U.S. Patent 4,589,876, "Shaped Sanitary Napkin With Flaps" issued to Van Tilburg on May 20, 1986.

Figure 1 shows an embodiment of the second absorbent article 100 in which the topsheet 124 and the backsheet 126 have length and width dimensions generally larger than those of the absorbent core 128. The topsheet 124 and the backsheet 126 extend beyond the edges of the absorbent core 128 to form portions of the periphery 140.

The absorbent core 128 may be any absorbent means which is capable of absorbing or retaining liquids (e.g., menses and/or urine). As shown in Figure 1 the absorbent core 128 has a body surface, a garment surface, side edges, and end edges. The absorbent core 128 may be manufactured in a wide variety of sizes and shapes (e.g., rectangular, oval, hourglass, dog bone, asymmetric, etc.) and from a wide variety of liquid-absorbent materials commonly used in various absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these. The configuration and construction of the absorbent core 128 may also be varied (e.g., the absorbent core 128 may have varying caliper zones (e.g., profiled so as to be thicker in the center), hydrophilic gradients, superabsorbent gradients, or lower density and lower average basis weight acquisition zones; or may comprise one or more layers or structures). The total absorbent capacity of the absorbent core 128 should, however, be compatible with the design loading and the intended use of the second absorbent article 100. Further, the size and absorbent capacity of the absorbent core 128 may be varied to accommodate different uses such as incontinence pads, pantiliners, regular sanitary napkins, or overnight sanitary napkins. The absorbent capacity of the absorbent core 128 may be the same as, greater than, or less than the absorbent capacity of the absorbent core 28 of the first absorbent article 20.

Exemplary absorbent structures for use as the absorbent core 128 of the present invention are described in U.S. Patent 4,950,264 entitled "Thin, Flexible Sanitary Napkin" issued to Osborn on August 21, 1990; U.S. Patent 4,610,678 entitled "High-Density Absorbent Structures" issued to Weisman et al. on September 9, 1986; U.S. Patent 4,834,735 entitled "High Density Absorbent Members Having Lower Density and Lower Basis Weight Acquisition Zones", issued to Alemany et al. on May 30, 1989; and European Patent Application No. 0 198 683, The Procter & Gamble Company, published October 22, 1986 in the name of Duenk, et al.

The backsheet 126 and the topsheet 124 are positioned adjacent the garment surface and the body surface, respectively, of the absorbent core 128 and are preferably joined thereto and to each other by attachment means (not shown) such as those well known in the art. For example, the backsheet 126 and/or the topsheet 124 may be secured to the absorbent core 128 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. The attachment means will preferably comprise an open pattern network of filaments of adhesive as is disclosed in U.S. Patent 4,573,986 entitled "Disposable Waste-Containment Garment", which issued to Minetola, et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague.. on October 7, 1975; U.S. Patent 4,785,996 issued to Ziecker, et al. on November 22, 1978; and U.S. Patent 4,842,666 issued to Werenicz on June 27, 1989.

Alternatively, the attachment means may comprise heat bonds, pressure bonds, ultrasonic bonds, dynamic mechanical bonds, or any other suitable attachment means or combinations of these attachment means as are known in the art.

The backsheet 126 is impervious to liquids (e.g., menses and/or urine) and is preferably manufactured from a thin plastic film, although other flexible liquid impervious materials may also be used. The backsheet 126 prevents the exudates absorbed and contained in the absorbent core 128 from wetting articles which contact the second absorbent article 100 such as pants, pajamas and undergarments. The backsheet 126 may thus comprise a woven or nonwoven material, polymeric films such as thermoplastic films of polyethylene or polypropylene, or composite materials such as a film-coated nonwoven material. Preferably, the backsheet 126 is a polyethylene film having a thickness of from about 0.012 mm (0.5 mil) to about 0.051 mm (2.0 mils). Exemplary polyethylene films are manufactured by Clopay Corporation of Cincinnati, Ohio. under the designation P18-0401 and by Ethyl Corporation, Visqueen Division, of Terre Haute, Indiana, under the designation XP-39385. The backsheet 126 is preferably embossed and/or matte finished to provide a more clothlike appearance. Further, the backsheet 126 may permit vapors to escape from the absorbent core 128 (i.e., breathable) while still preventing exudates from passing through the backsheet 126.

The topsheet 124 is compliant, soft feeling, and non-irritating to the wearer's skin. Further, the topsheet 124 is liquid pervious permitting liquids (e.g., menses and/or urine) to readily penetrate through its thickness. A suitable topsheet 124 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. A preferred topsheet 124 comprises an apertured formed film. Apertured formed films are preferred for the topsheet 124 because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are the same as those disclosed herein for the topsheet 24, and each of the patents discussed for the topsheet 24 are hereby incorporated by reference for topsheet 124. The preferred topsheet 124 for the present invention is the formed film described in one or more of the patents described for topsheet 24 and marketed on sanitary napkins by The Procter & Gamble Company of Cincinnati, Ohio as "DRI-WEAVE".

In a preferred embodiment of the present invention, the body surface of the formed film topsheet is hydrophilic so as to help liquid to transfer through the topsheet faster than if the body surface was not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in PCT Patent Application WO 93/09741 "Absorbent Article Having A Nonwoven and Apertured Film Coversheet". Alternatively, the body surface of the topsheet can be made hydrophilic by treating it with a surfactant such as is described in the above referenced U.S. 4,950,264 issued to Osborn.

In a preferred embodiment of the present invention, an acquisition layer(s) may be positioned between the topsheets and the absorbent cores of the first absorbent article 20 and the second absorbent article 100, respectively. An acquisition layer may serve several functions including improving wicking of exudates over and into the absorbent core. There are several reasons why the improved wicking of exudates is important, including providing a more even distribution of the exudates throughout an absorbent core and allowing an absorbent article described herein to be made relatively thin. (The wicking referred to herein may encompass the transportation of liquids in one, two or all directions (i.e., in the x-y plane and/or in the z-direction). The acquisition layer may be comprised of several different materials including nonwoven or woven webs of synthetic fibers including polyester, polypropylene, or polyethylene; natural fibers including cotton or cellulose; blends of such fibers; or any equivalent materials or combinations of materials Examples of absorbent articles disclosed herein having an acquisition layer and a topsheet are more fully described in U.S. 4,950,264 issued to Osborn. In a preferred embodiment, the acquisition layer may be joined with the topsheet by any of the conventional means for joining webs together, most preferably by fusion bonds.

To form the combination absorbent article 10 of the present invention, the first absorbent article 20 and the second absorbent article 100 are releasably secured together only by mechanical securement means, thereby providing a mechanical engagement between the first absorbent article 20 and the second absorbent article 100. The precise nature of the mechanical securement means is immaterial so long as the mechanical securement means serves to releasably secure the absorbent articles to one another by mechanical engagement such that the absorbent articles are secured to one another until separated by the user. As shown in Fig. 1, the first and second absorbent articles 20 and 100 are releasably secured together by mechanical securement means 38. In this embodiment not forming part of the present invention, the longitudinal edges 30 and 130, and the end edges 35 and 135 of the first and second absorbent articles 20 and 100, respectively, are releasably secured together by mechanical securement means 38.

The mechanical securement means may comprise crimps. In such a process, a first absorbent article is formed having a topsheet, a backsheet joined to the topsheet, and an absorbent core positioned between the topsheet and the backsheet. The formed first absorbent article further comprises a body surface, a garment surface, and a periphery comprising a pair of first longitudinal edges and a pair of first end edges. A second absorbent article is formed and comprises a topsheet, a backsheet joined to the topsheet, an absorbent core positioned between the topsheet and backsheet, an article contacting surface, a garment surface, and a periphery comprising a pair of second longitudinal edges and a pair of second end edges.

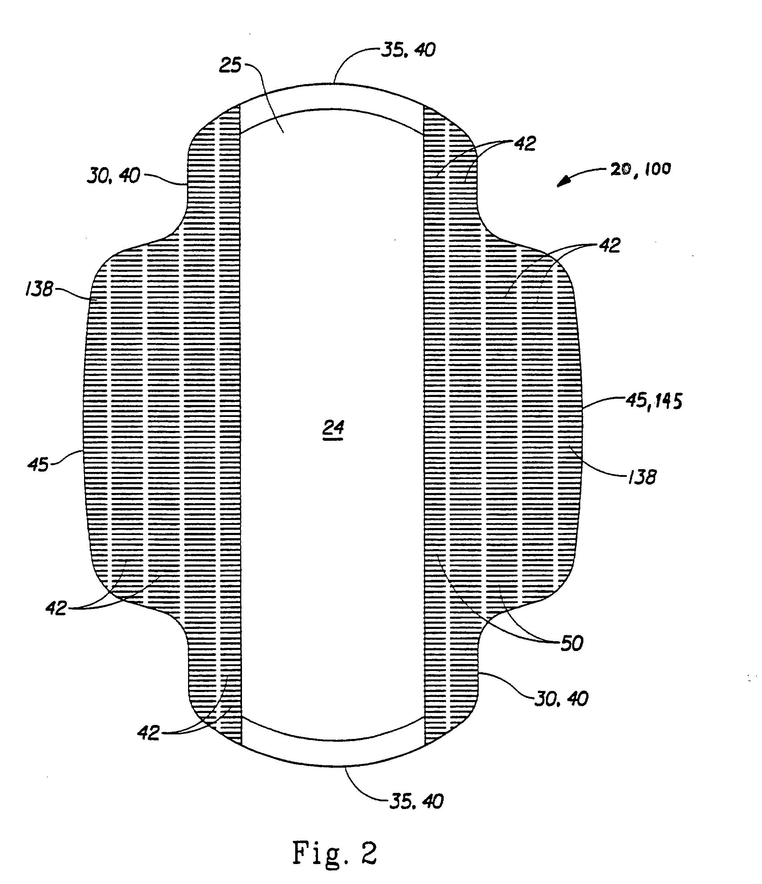

After forming the first and second absorbent articles, the first absorbent article is laid onto the second absorbent article such that the peripheries of the articles are substantially aligned and symmetrically correct. By the term "symmetrically correct" it is meant herein that the first absorbent article will lay on the second absorbent article such that when looking at the combination absorbent article from the top or directly overhead, only one absorbent article is seen, e.g., the top view in Fig. 2.

Next, the first and second absorbent articles are delivered together in a symmetrically correct fashion to at least one crimping/embossment means such as a mating embossment roll. By "symmetrically correct fashion" it is meant herein that the first and second absorbent articles are delivered one on top of the other in a back to face configuration (i.e., symmetrically correct) without substantial variance in their positions. To finish, the first and second absorbent articles are embossed/crimped together along their longitudinal edges and in an alternative embodiment, along their end edges. Of course, another possible embodiment is one in which the longitudinal edges 30 and 130 and at least a portion of the end edges 35 and 135 are embossed/crimped together to provide mechanical securement.

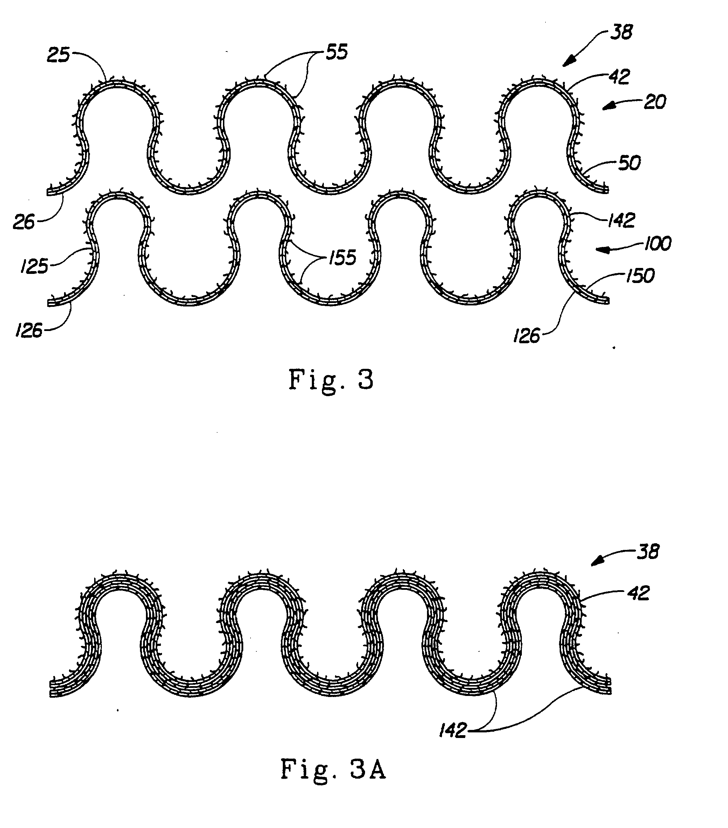

Figures 3 and 3A provide enlarged side views of a preferred mechanical securement means 38 in which crimps or embossments 42 in first absorbent article 20 and crimps 142 in second absorbent article 100 are shown to be separated from one another for illustrative purposes only. Figure 3A shows crimps 42 and 142 being in operative relationship to one-another as they would be in a mechanically engaged absorbent article. In practice, the crimps 42 and 142 that are present about the peripheries 40 and 140 (Fig. 1), respectively, can be joined together by two or more mating crimp/embossment rolls. At such joining the crimps 42 and 142 are mechanically engaged with one another without the use of any adhesive. In this state the absorbent articles 20 and 100 will remain mechanically engaged with one another during the normal wear of the combination absorbent article 10 (Fig. 1). However, when the first absorbent article 20 has been soiled and requires disposal, it can be readily detached from the second absorbent article 100 by pulling the first absorbent article 20 at one end edge 35 to the other end edge 35, thus completely disrupting the mechanical engagement between the first and second.absorbent articles 20 and 100, respectively.

In a preferred embodiment shown in Figs. 2 and 3, the first and second absorbent articles 20 and 100 will each comprise a nonwoven web or fabric 50 and 150, respectively, that when added to the first and second absorbent articles, make-up the body-contacting surfaces 25 and 125 (Fig. 1) of each respective article. Note, when the first absorbent article 20 is detached from the second absorbent article 100, the article-contacting surface 125 then becomes the body-contacting surface 125; for all purposes herein, these two terms are interchangeable for the second absorbent article 100. These layers function to provide increased comfort to a user's skin, some absorbency and importantly, increased cohesion between the crimps 42 and 142 of the first and second absorbent articles 20 and 100 when they are mechanically secured together about their peripheries 40 and 140 and flaps 45 and 145.

The nonwoven webs 50 and 150 comprise fibrous elements or fibers 55 and 155 that project outwardly in numerous directions from the nonwoven webs as shown in Fig. 3. Note, however, that the fibrous elements can comprise varying lengths and many projection directions extending from the nonwoven layer. While not wishing to be bound by any particular theory, it is believed that the projecting fibrous elements 55 and 155 of the nonwoven webs 50 and 150 provide increased cohesion through frictional forces exerted between the fibrous elements 155 and the backsheet 26 of the first absorbent article 20. Thus, the fibrous elements 155 on the second absorbent article 100 interact with the polymer backsheet 26 of the first absorbent article 20 in such a way as to provide a sufficient amount of cohesive strength between the articles when their peripheries and flaps are crimped together.

In using mechanical engagement versus adhesive, several problems are solved. For example, there is no possibility of adhesive exposure to a user's skin and other sensitive areas of the user's body. This is important because an adhesive can be contaminated with foreign objects and thus become unsanitary. Also, adhesive can irritate a user by becoming entangled with a user's pubic hair during wear of the combination absorbent article. When using adhesive that lies between the garment facing surface of a first absorbent article and the body facing surface of a second absorbent article, the possibility of residual adhesive being on the body facing surface of the second absorbent article exists when the first absorbent article is pulled from it. Mechanical engagement avoids this possibility and ensures that the body facing surface of the second absorbent article will remain free from any adhesive. Thus Applicants' invention avoids possible irritation and/or infection associated with the use of adhesives.

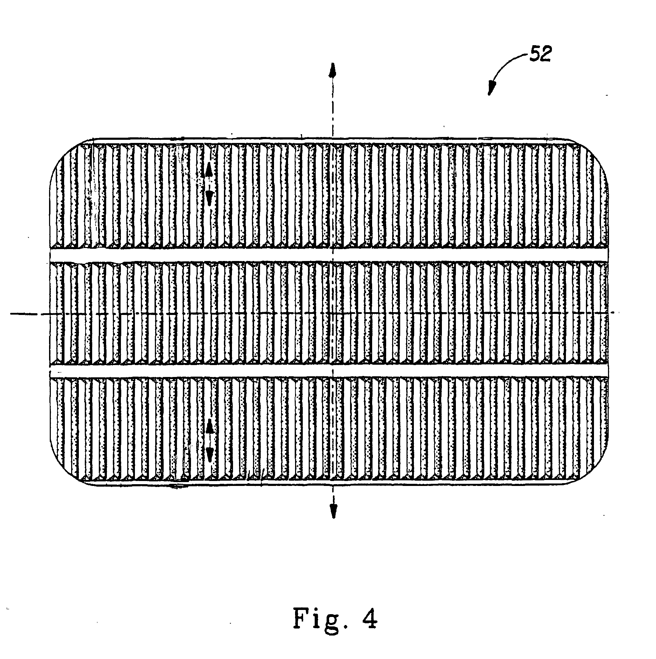

In a preferred embodiment, the mechanical securement means 38 comprises the embossment pattern of a structural elastic-like film (SELF) web about the peripheries 40 and 140 and/or flaps if present. The SELF embossment pattern is the primary mechanical securement means for releasably securing the first and second absorbent articles together. Figure 4 shows an embodiment of a SELF embossment pattern 52 of the present invention. The SELF embossment pattern is discussed in greater detail in U.S. Patent No. 5,518.801 issued May 21, 1996, in the name of Chappell et al.

In Figure 2, there is shown the embodiment of the present invention. The first absorbent article 20 is shown comprising a pair of flaps 45, each of which are adjacent to and extend laterally from the longitudinal edges 30 and 130 of the absorbent core 28 and 128. The second absorbent article 100, comprising flaps 145 resides beneath the first absorbent article 20. The two absorbent articles 20 and 100 would be secured not only about their peripheries 40 and 140 but also about their flaps 45 and 145. The flaps 45 are configured to drape over the edges of the wearer's panties in the crotch region so that the flaps 45 are disposed between the edges of the wearer's panties and the thighs. The flaps 45 and 145 serve at least one purpose. For example, the flaps 45 and 145 help serve to prevent soiling of the wearer's body and panties by menstrual fluid, preferably by forming a double wall barrier along the edges of the panty. Additionally, the flaps 145 are preferably provided with attachment means, preferably being adhesive, on their garment surface so that they can fold back under the panty and attach to the garment facing side of the panty or attach one flap to another. The flaps 45 and 145 can be constructed of various materials including materials similar to the topsheet, backsheet, tissue, or combination of these materials. Further, the flaps 45 and 145 may be a separate element attached respectively to the main body of the first absorbent article 20 and second absorbent article 100 or can comprise extensions of their respective topsheets and backsheets (i.e., a unitary construction). A number of sanitary napkins having flaps 45 suitable or adaptable for use with the sanitary napkins of the present invention are disclosed in U.S. 4,687,478 entitled "Shaped Sanitary Napkin With Flaps", which issued to Van Tilburg on August 18, 1987; U.S. 4,589,876 entitled "Sanitary Napkin", which issued to Van Tilburg on May 20, 1986; and U.S. 4,608,047, entitled "Sanitary Napkin Attachment Means", which issued to Mattingly on August 26, 1986.

Like the peripheries 40 and 140 of previous embodiments disclosed herein, the flaps 45 and 145 are preferably embossed together to form mechanical securement means 138 at the flaps 45 and 145. Most preferably, the flaps 45 and 145 also comprise nonwoven webs 50 and 150 that have been extended from peripheries 40 and 140. Note, the crimping of the flaps 45 and 145 and the peripheries 40 and 140 is preferably the same as previously disclosed herein and is performed in one or more simultaneous embossment processes (e.g., the SELF embossment process as discussed in U.S. Patent No. 5,518,801 issued May 21, 1996, in the name of Chappell et al.).

Additionally, other mechanical securement means may be utilized as should be well known by one skilled in the art. Further, mechanical entanglement releasable securement means, such as needle punching, or nearly any of the various types of embossing may be utilized with the present invention as is well known by one skilled in the art.

It will be readily apparent to one skilled in the art that many variations are feasible. For applicable materials known in the art for such, the first and second longitudinal edges 30 and 130 may be wetted with a solution. Suitable wetting solutions are disclosed in the aforementioned U.S. Patent No. 5,332,118 issued July 26, 1994 to Muckenfuhs. While not wishing to bound by any particular theory, it is believed that wetting will enable the edges 30 and 130 made from a suitable material herein to be releasably attached to one another through cohesion at the overlap between adjacent tissues. The cohesion may not require any other manufacturing step other than to meet the first longitudinal edges 30 with the second longitudinal edges 130. Additionally, such cohesion may not require any type of intermediary, e.g., a liquid, to provide mechanical securement to the longitudinal and/or end edges. For example, such cohesion can be observed when one folds a piece of polyvinylidene chloride film (i.e., SARANWRAP plastic marketed by the Dow Chemical Corporation) onto itself. At just the touching of such films, a cohesive bond is instantly asserted and maintained.

In an alternative embodiment, the present invention provides an absorbent article, such as a sanitary napkin, that disperses into fragments which are readily flushable in a normal toilet. The preferred sanitary napkin of the present invention comprises a liquid pervious topsheet, a backsheet impervious to bodily fluids, an absorbent core positioned between the topsheet and the backsheet, and means for removably attaching the sanitary napkin to a wearer's undergarment. Additionally, embodiments of the present invention can also comprise laterally extending flaps.

The preferred liquid pervious topsheet of the present alternative embodiment comprises a wet laid apertured tissue having a temporary wet strength resin incorporated therein. Portions of the body surface of the tissue are further provided with a resinous material. Preferably, the resinous material comprises a water resistant resinous material that is provided in the form of fibrils printed on the body surface of the topsheet. Alternatively, the resinous material can provide the topsheet with a surface energy gradient between the body surface thereof and the garment surface thereof. The preferred topsheet of the present invention acquires bodily fluids at an excellent rate and serves to prevent such acquired fluids from rewetting the body surface thereof so the sanitary napkin of the present invention has a comfortable feel when it is worn.

In an alternative embodiment, the first and second backsheets of the present invention each comprise a wet laid fibrous assembly having a temporary wet strength resin incorporated therein. The backsheets are further coated with a water resistant resinous material that causes the backsheets to become impervious to bodily fluids without impairing the spreading of adhesive materials thereon. Backsheets of the type described herein represent an improvement over those described in the art in that flushable absorbent articles of the prior art typically use materials having a very low critical surface tension to help ensure the backsheets to be impervious with resulting difficulty in adhesively joining such backsheets to the remaining components of an absorbent article. The backsheets-of the present embodiment presents no such joinder issues.

The absorbent articles are assembled by disposing each backsheet of the combination absorbent article herein such that the surface thereof that is coated with the water resistant resinous material is oriented toward each core. Each core and topsheet are disposed thereon, and the components joined using means known to those skilled in the art. A water soluble adhesive is used to join the components of each sanitary napkin in the combination in at least an area of peripheral bonding so the components will separate when either a first or a second absorbent article is exposed to water in a toilet.

Once the flushable absorbent articles are formed, the first absorbent article is laid onto the second absorbent article such that the peripheries of the articles are substantially aligned and symmetrically correct. By the term "symmetrically correct" it is meant herein that the first absorbent article will lay on the second absorbent article such that when looking at the combination absorbent article from the top, only one absorbent article is seen, e.g., the top view in Fig. 2.

Next, the first and second absorbent articles are delivered together in a symmetrically correct fashion to at least one crimping/embossment means such as a mating embossment roll. By "symmetrically correct fashion" it is meant herein that the first and second absorbent articles are delivered one on top of the other in a back to face configuration (i.e., symmetrically correct) without substantial variance in their positions. To finish, the first and second absorbent articles are embossed/crimped together along their longitudinal edges and flaps and in an alternative embodiment, also along their end edges. Flushable absorbent articles are described in greater detail in U.S. Patent US 5722966, entitled "Water Dispersible and Flushable Absorbent Article" filed on November 22, 1995 by Christon, et al.

In use, the combination absorbent article can be held in place by any support means or attachment means (not shown) well-known for such purposes. Preferably, the combination absorbent article is placed in the user's undergarment or panty and secured thereto by a fastener such as an adhesive. The adhesive provides a means for securing the combination absorbent article in the crotch portion of the panty. Thus, a portion or all of the outer surface of the backsheet 126 of the second absorbent article 100 is coated with adhesive. Any adhesive or glue used in the art for such purposes can be used for the adhesive herein, with pressure-sensitive adhesives being preferred. Suitable adhesives are Century A-305-IV manufactured by the Century Adhesives Corporation of Columbus, Ohio; and Instant Lock 34-2823 manufactured by the National Starch and Chemical Company of Bridgewater, NI. Suitable adhesive fasteners are also described in U.S. Patent 4,917,697. Before the combination absorbent article is placed in use, the pressure-sensitive adhesive is typically covered with a removable release liner in order to keep the adhesive from drying out or adhering to a surface other than the crotch portion of the panty prior to use. Suitable release liners are also described in the above-referenced U.S. Patent 4,917,697. Any commercially available release liners commonly used for such purposes can be utilized herein. Non-limiting examples of suitable release liners are BL30MG-A Silox E1/0 and BL30MG-A Silox 4P/O both of which are manufactured by the Akrosil Corporation of Menasha, WI. The combination absorbent article of the present invention is used by removing the release liner and thereafter placing the combination absorbent article in a panty so that the adhesive contacts the panty. The adhesive maintains the combination absorbent article in its position within the panty during use.

## Claims

1. A combination absorbent article (10) having a first absorbent article (20) comprising a body-contacting surface (25), a garment surface (27), an absorbent core (28), a liquid impervious backsheet (26), and a periphery (40) comprising a pair of first longitudinal edges (30) and a pair of first end edges (35), and a second absorbent article (100) comprising an article contacting surface (125), a garment surface (127), an absorbent core (128), a liquid impervious backsheet (126), and a periphery (140) comprising a pair of second longitudinal edges (130) and a pair of second end edges (135).
said peripheries (40, 140) of said first absorbent article (20) and said second absorbent article (100) being symmetrically correct, and wherein
said first longitudinal edges (30) of said first absorbent article (20) and said second longitudinal edges (130) of said second absorbent article (100) are releasably secured together only by mechanical securement means (38),
said combination absorbent article (10) further comprising a pair of flaps (45) joined to said first absorbent article (20) and a pair of flaps (145) joined to said second absorbent article (100), said flaps being disposed adjacent to and extending laterally outwardly from each said first and second longitudinal edges, thereby having at least one of said flaps being joined adjacent to each said longitudinal edge,
said combination absorbent article (10) **characterized in that** each said flap (45, 145) comprises mechanical securement means (138) whereby said flaps on said first (20) and second (100) absorbent articles are releasably secured together.

2. The combination absorbent article (10) of Claim 1 wherein said article contacting surface (125) of said second absorbent article (100) comprises a nonwoven layer (150).

3. The combination absorbent article (10) of any one of the preceding claims wherein said mechanical securement means (38,138) comprises a plurality of crimps (42,142).

4. The combination absorbent article (10) of any one of the preceding claims wherein said first end edges (35) of said first absorbent article (20) and said second end edges (135) of said second absorbent article (20) are releasably secured together only by mechanical securement means (38).

5. The combination absorbent article (10) of Claim 4 wherein said mechanical securement means (38) comprises a plurality of crimps.

## Patentansprüche

1. Kombinierter absorbierender Artikel (10), der einen ersten absorbierenden Artikel (20) mit einer körperberührenden Oberfläche (25), einer wäscheseitigen Oberfläche (27), einem absorbierenden Kern (28), einer flüssigkeitsundurchlässigen Unterschicht (26) und einem Umfang (40) mit einem Paar erster Längsränder (30) und einem Paar erster Stirnränder (35) und einen zweiten absorbierenden Artikel (100) mit einer artikelberührenden Oberfläche (125), einer wäscheseitigen Oberfläche (127), einem absorbierenden Kern (128), einer flüssigkeitsundurchlässigen Unterschicht (126) und einem Umfang (140) mit einem Paar zweiter Längsränder (130) und einem Paar zweiter Stirnränder (135) aufweist,
wobei die Umfänge (40, 140) des ersten absorbierenden Artikels (20) und des zweiten absorbierenden Artikels (100) symmetrisch korrekt sind, und in welchem
die ersten Längsränder (30) des ersten absorbierenden Artikels (20) und die zweiten Längsränder (130) des zweiten absorbierenden Artikels (100) nur durch mechanische Befestigungsmittel (38) lösbar aneinander festgelegt sind,
wobei der kombinierte absorbierende Artikel (10) ferner aufweist ein Paar Klappen (45), die mit dem ersten absorbierenden Artikel (20) verbunden sind, und ein Paar Klappen (145), die mit dem zweiten absorbierenden Artikel (100) verbunden sind,
wobei die Klappen angrenzend an dem ersten und zweiten Längsrand und sich seitlich von jedem derselben nach außen erstreckend angeordnet sind, wodurch wenigstens eine der Klappen an jedem Längsrand verbunden ist,
wobei der kombinierte absorbierende Artikel (10) **dadurch gekennzeichnet ist, daß** jede Klappe (45, 145) ein mechanisches Befestigungsmittel (138) aufweist, wodurch die Klappen auf dem ersten (20) und dem zweiten (100) absorbierenden Artikel lösbar aneinander festgelegt sind.

2. Kombinierter absorbierender Artikel (10) nach Anspruch 1, in welchem die artikelberührende Oberfläche (125) des zweiten absorbierenden Artikels (100) eine Vliesschicht (150) aufweist.

3. Kombinierter absorbierender Artikel (10) nach einem der vorstehenden Ansprüche, in welchem das mechanische Befestigungsmittel (38, 138) eine Mehrzahl Fältelungen (42, 142) aufweist.

4. Kombinierter absorbierender Artikel (10) nach einem der vorstehenden Ansprüche, in welchem die ersten Stirnränder (35) des ersten absorbierenden Artikels (20) und die zweiten Stirnränder (135) des zweiten absorbierenden Artikels (20) nur durch mechanische Befestigungsmittel (38) lösbar aneinander festgelegt sind.

5. Kombinierter absorbierender Artikel (10) nach Anspruch 4, in welchem das mechanische Befestigungsmittel (38) eine Mehrzahl von Fältelungen aufweist.

## Revendications

1. Article absorbant combiné (10) comportant un premier article absorbant (20) comprenant une surface en contact avec le corps (25), une surface de vêtement (27), une âme absorbante (28), une feuille de fond (26) imperméable aux liquides et une périphérie (40) comprenant une paire de premiers bords longitudinaux (30) et une paire de premiers bords d'extrémité (35), et un deuxième article absorbant (100) comprenant une surface en contact avec l'article (125), une surface de vêtement (127), une âme absorbante (128), une feuille de fond (126) imperméable aux liquides et une périphérie (140) comprenant une paire de deuxièmes bords longitudinaux (130) et une paire de deuxièmes bords d'extrémité (135),
lesdites périphéries (40, 140) dudit premier article absorbant (20) et dudit deuxième article absorbant (100) étant symétriquement correctes, et dans lequel
lesdits premiers bords longitudinaux (30) dudit premier article absorbant (20) et lesdits deuxièmes bords longitudinaux (130) dudit deuxième article absorbant (100) ne sont assujettis ensemble de manière libérable que par un moyen d'assujettissement mécanique (38),
ledit article absorbant combiné (10) comprenant, en outre, une paire de rabats (45) réunis audit premier article absorbant (20) et une paire de rabats (145) réunis audit deuxième article absorbant (100), lesdits rabats étant placés à proximité immédiate de chaque dit premier et deuxième bord longitudinal et s'étendant latéralement vers l'extérieur à partir de chaque dit premier et deuxième bord longitudinal, au moins un desdits rabats étant, ainsi, réuni de manière adjacente à chaque dit bord longitudinal,
ledit article absorbant combiné (10) étant **caractérisé en ce que** chaque dit rabat (45, 145) comprend un moyen d'assujettissement mécanique (138), de sorte que lesdits rabats situés sur chaque dit premier (20) et deuxième (100) article absorbant sont assujettis ensemble de manière libérable.

2. Article absorbant combiné (10) selon la revendication 1, dans lequel ladite surface en contact avec l'article (125) dudit deuxième article absorbant (100) comprend une couche non tissée (150).

3. Article absorbant combiné (10) selon l'une quelconque des revendications précédentes, dans lequel ledit moyen d'assujettissement mécanique (38, 138) comprend une pluralité de gaufrages (42, 142).

4. Article absorbant combiné (10) selon l'une quelconque des revendications précédentes, dans lequel lesdits premiers bords d'extrémité (35) dudit premier article absorbant (20) et lesdits deuxièmes bords d'extrémité (135) dudit deuxième article absorbant (20) ne sont assujettis ensemble de manière libérable que par un moyen d'assujettissement mécanique (38).

5. Article absorbant combiné (10) selon la revendication 4, dans lequel ledit moyen d'assujettissement mécanique (38) comprend une pluralité de gaufrages.
